Europäisches Patentamt

European Patent Office

Office européen des brevets

(19)

(11) **EP 1 242 357 B1**

(12) **FASCICULE DE BREVET EUROPEEN**

(45) Date de publication et mention
de la délivrance du brevet:
**21.07.2004 Bulletin 2004/30**

(51) Int Cl.7: **C07C 67/08**, C07C 67/03,
C07C 69/68

(21) Numéro de dépôt: **00993606.3**

(22) Date de dépôt: **26.12.2000**

(86) Numéro de dépôt international:
**PCT/FR2000/003685**

(87) Numéro de publication internationale:
**WO 2001/047860 (05.07.2001 Gazette 2001/27)**

(54) **PROCEDE DE PREPARATION D'UNE COMPOSITION D'ESTER D'ACIDE LACTIQUE ET SON UTILISATION EN TANT QUE SOLVANT**

VERFAHREN ZUR HERSTELLUNG EINER MILCHSÄUREESTER-ZUSAMMENSETZUNG UND DEREN GEBRAUCH ALS LÖSUNGSMITTEL

METHOD FOR PREPARING A LACTIC ACID ESTER COMPOSITION AND USE THEREOF AS SOLVENT

(84) Etats contractants désignés:
**AT BE CH CY DE DK ES FI FR GB GR IE IT LI LU MC NL PT SE TR**

(30) Priorité: **28.12.1999 FR 9916608**

(43) Date de publication de la demande:
**25.09.2002 Bulletin 2002/39**

(73) Titulaire: **Roquette Frères**
**62136 Lestrem (FR)**

(72) Inventeurs:
• **FUERTES, Patrick**
**F-59130 Lambersart (FR)**
• **TAMION, Rodolphe**
**F-62157 Allouagne (FR)**

• **FLECHE, Guy**
**F-59190 Hazebrouck (FR)**
• **COMINI, Serge**
**F-59253 La Gorgue (FR)**

(74) Mandataire: **Boulinguiez, Didier et al**
**Cabinet Plasseraud**
**65/67 rue de la Victoire**
**75440 Paris Cedex 09 (FR)**

(56) Documents cités:
**EP-A- 0 628 533    US-A- 5 264 617**

• **DATABASE WPI Week 200003 Derwent Publications Ltd., London, GB; AN 2000-024264 XP002146865 & CN 1 229 790 A (YIN C), 29 septembre 1999 (1999-09-29)**

EP 1 242 357 B1

**Description**

La présente invention a pour objet un nouveau procédé de préparation d'une composition d'ester d'acide lactique à partir d'une composition d'acide lactique, ledit procédé pouvant avantageusement être mené en continu. Elle vise également l'utilisation de la composition d'ester d'acide lactique ainsi obtenue en tant que solvant utile, entre autres, à la préparation de compositions gélifiées.

L'usage industriel de solvants tels que les solvants chlorés, les glycols et éthers de glycols, est de plus en plus remis en cause du fait de la toxicité de ces produits et de leur impact négatif sur l'environnement. Il est donc recherché de les substituer par des solvants dits "verts", à savoir biodégradables et non toxiques, tels que les esters d'acide lactique et en particulier le lactate d'éthyle. Celui-ci présente un certain nombre de propriétés physico-chimiques l'autorisant, au moins potentiellement, à se substituer à tout ou partie des solvants classiques et, entre autres :

- un excellent pouvoir solvant des résines (nitrocellulose, résines acryliques, polyuréthannes, polyesters, alkydes, époxy, ...),
- une solubilité relativement élevée dans l'eau et généralement bonne dans la plupart des solvants organiques,
- une volatilité relativement peu élevée autorisant, par exemple, son emploi efficace dans les applications de traitement de surface,
- un point d'ébullition relativement élevé (154°C) autorisant son emploi efficace dans les applications impliquant des hautes températures.

Les propriétés de solvant du lactate d'éthyle et autres esters d'acide lactique peuvent avantageusement être mises à profit dans de nombreux domaines d'activité tels que les industries des métaux, les industries de l'automobile et de l'aviation, les industries des peintures et encres, les industries des résines et laques, les industries pharmaceutiques et cosmétologiques et les industries des semi-conducteurs.

Il convient de préciser que la notion de solvant n'est ici aucunement limitative et inclut, en particulier, les notions d'agent co-solvant, nettoyant, dégraissant ou décapant.

Ces esters d'acide lactique, et en particulier les lactates d'éthyle ou de butyle, trouvent cependant d'autres usages industriels et ce, en tant qu'intermédiaires de synthèse, notamment dans la préparation :

- d'acide lactique de très haute pureté comme décrit, par exemple, dans les brevets US 5,210,296 et EP 614 983 ou,
- de lactide, i.e de dimère cyclique d'acide lactique, puis de polylactides comme décrit, par exemple, dans le brevet WO 93/15127.

Malgré son intérêt indéniable comme solvant ou intermédiaire de synthèse, un produit comme le lactate d'éthyle reste à ce jour encore peu fabriqué et utilisé industriellement, notamment en regard des solvants chlorés ou des (éthers de) glycols. Ceci, du fait de son prix qui, à titre d'exemple, est actuellement encore deux à quatre fois plus élevé que celui des principaux éthers de glycols accessibles sur le marché.

Il est largement connu que la difficulté de préparation industrielle du lactate d'éthyle à un coût économiquement acceptable est notamment liée à la double contrainte technique 1) de devoir éliminer, en continu, l'eau présente dans le milieu réactionnel aux fins de déplacer l'équilibre de ladite réaction dans le sens souhaité et 2) de devoir mettre en oeuvre des quantités d'alcool, en l'occurrence d'éthanol, largement excédentaires en regard de celles théoriquement nécessaires à la réaction d'estérification de l'acide lactique.

Cette contrainte est d'autant plus accentuée que l'éthanol présente, en regard d'autres alcools comme le butanol, un azéotrope peu riche en eau rendant l'élimination de l'eau forcément coûteuse.

En effet, certaines technologies de préparation d'esters d'acide lactique sont considérées comme non applicables à la production spécifique du lactate d'éthyle. C'est le cas, par exemple, des procédés exemplifiés dans les brevets US 5,210,296 et EP 614 983 précités, décrivant la préparation de lactate de butyle après mise en oeuvre de n-butanol dans un milieu de culture, éventuellement concentré et/ou purifié, ayant généré du lactate d'ammonium.

Selon les auteurs de ces brevets, les alcools à 4 ou 5 atomes de carbone sont ceux, parmi les alcools utilisables pour estérifier l'acide lactique, qui présentent le meilleur compromis économique global en termes de rendements de formation puis de purification d'esters mais également d'hydrolyse subséquente éventuelle desdits esters en vue de récupérer de l'acide lactique de haute pureté. Le n-butanol est ainsi décrit dans le brevet US 5,210,296 comme présentant les avantages technico-économiques suivants :

- point d'ébullition (117,7°C) très significativement supérieur à celui de l'eau et très significativement inférieur à celui du lactate de butyle (187°C) ,
- faible miscibilité à l'eau et possibilité de former des azéotropes hétérogènes avec l'eau,
- possibilité d'être distillé sans donner lieu à des problèmes a) de polymérisation ou dimérisation du lactate de butyle, b) d'hydrolyse du lactate de butyle et c) d'augmentation de la viscosité du milieu réactionnel.

Il a été envisagé récemment, dans le brevet US 5,723,639, de perfectionner les procédés susmentionnés de préparation d'esters d'acide lactique à partir de lactate d'ammonium et notamment de pouvoir rendre lesdits procédés véritablement applicables à la préparation du lactate d'éthyle et/ou à l'usage de températures réactionnelles limitées, i.e inférieures à 100°C. Ledit brevet revendique à cette fin la mise en oeuvre obligatoire d'une étape de pervaporation du milieu d'estérification en vue d'en éliminer à la fois l'eau et l'ammoniaque et ce, sans élimination concommittante de l'alcool (éthanol, butanol) ou de l'ester formé (lactate d'éthyle ou de butyle).

Cette technologie impose cependant la mise en oeuvre de dispositifs de pervaporation relativement spécifiques et coûteux et en particulier de membranes de pervaporation devant présenter, mais surtout conserver au cours du temps, des caractéristiques très particulières de sélectivité et de résistance aux conditions de milieu (températures comprises entre 75 et 150°C, présence de composés acides ou alcalins, ...).

En outre, si la possibilité de travailler à des températures relativement basses (80-95°C par exemple) permet de limiter le taux d'éthanol mis en oeuvre à une valeur de 2 moles/mole d'acide lactique comme décrit dans les exemples 6 et 7 dudit brevet, cette possibilité n'engendre pas moins en pratique un certain nombre d'effets néfastes à l'économie générale du procédé et notamment a) des taux d'introduction relativement élevés en catalyseurs d'estérification (généralement de 1 à 10 %) et/ou b) des durées réactionnelles très importantes (généralement de plusieurs dizaines d'heures) et/ou c) des taux de conversion de l'acide lactique (ou du lactate d'ammonium) en lactate d'éthyle, (très) peu élevés.

Il a également été proposé de préparer du lactate d'éthyle, non pas à partir d'acide lactique ou l'un de ses sels, mais à partir de composés très spécifiques tels que le lactide comme décrit dans le résumé du brevet japonais n° 8-40983 ou des polylactides de poids moléculaire élevé (200 000) comme décrit dans l'exemple 10 du brevet US 5,264,617.

De tels procédés ne sont pas économiquement viables puisqu'ils utilisent des matières premières (lactide, polylactides) encore plus coûteuses que le produit recherché (lactate d'éthyle). En effet, comme il a déjà été souligné, le lactate d'éthyle est lui-même utilisé en vue de la préparation de lactide et de polylactides, et il est économiquement inapproprié de vouloir retransformer ces produits en lactate d'éthyle, composé de plus faible valeur ajoutée.

En outre, selon l'exemple 10 du brevet US 5,264,617, le taux de conversion du polylactide en lactate d'éthyle reste limité puisque n'atteignant qu'une valeur de 78 %.

Il résulte de ce qui précède qu'il existe un besoin de disposer d'un moyen simple, efficace et moins coûteux que ceux précités, d'obtention d'esters d'acide lactique et en particulier de lactate d'éthyle, moyen qui permette notamment :

- d'utiliser l'acide lactique, purifié ou non, comme matière première,

- de limiter les problèmes techniques et économiques liés à la présence et à l'élimination de l'eau dans le milieu d'estérification,

- de limiter les problèmes techniques et économiques liés à la mise en oeuvre et à la récupération des alcools, en particulier de l'éthanol, dans le milieu d'estérification,

- d'estérifier l'acide lactique à des températures relativement élevées, dans des durées réactionnelles relativement courtes et/ou sans devoir surdoser les catalyseurs d'estérification, et

- de préparer lesdits esters, en particulier le lactate d'éthyle, avec des taux de conversion élevés, i.e supérieurs à 85 % et ce, sans mise en oeuvre d'équipements spécifiques, complexes ou coûteux.

Et la Société Demanderesse a trouvé, après de nombreuses recherches, qu'un tel moyen pouvait consister en la transformation, avant son estérification par un alcool, de l'acide lactique en une composition intermédiaire particulière, de nature oligomérique.

De façon plus précise, la présente invention a pour objet un procédé de préparation d'une composition d'ester d'acide lactique à partir d'une composition d'acide lactique, ledit procédé étant caractérisé par le fait qu'il comprend :

a) une étape de transformation, avec élimination d'eau, de ladite composition d'acide lactique en une composition oligomérique d'acide lactique présentant un degré de polymérisation moyen (DPM) compris entre 2 et 30 environ,
b) une étape subséquente de mélange et de réaction de ladite composition oligomérique, purifiée ou non, avec un alcool, en présence d'un catalyseur de transestérification, pour estérifier tout ou partie de l'acide lactique contenu, sous forme monomérique, dimérique, oligomérique ou polymérique, dans ladite composition oligomérique, et
c) une étape éventuelle de purification de la composition d'ester d'acide lactique ainsi obtenue.

La transformation préalable de la composition d'acide lactique en une composition oligomérique telle que décrite présente comme avantage indéniable de limiter de manière très substantielle le besoin d'élimination d'eau lors de l'étape subséquente d'estérification et donc de minimiser voire d'annihiler les problèmes technico-économiques liés aux opérations de distillation azéotropique ou de pervaporation classiquement mises en oeuvre lors de ladite étape.

Par " composition d'acide lactique " au sens de la présente invention, on entend en particulier toute solution aqueuse d'acide lactique, quels que soient son procédé d'obtention et ses caractéristiques, ladite solution pouvant, par exemple, présenter une matière sèche (MS) et une pureté en acide lactique très variables. Il peut s'agir en particulier de solutions du commerce à 50, 80, 88 ou 90 % de matière sèche (MS) étant entendu, comme rappelé par exemple dans le brevet US 5,210,296 (cf colonne 1, lignes 50-57), que de telles solutions sont en fait des mélanges d'eau, de monomères, de dimères et d'oligomères d'acide lactique.

De telles solutions peuvent en outre présenter des taux variables d'impuretés. En suite de quoi, la composition d'acide lactique utilisable comme matière première dans le cadre de l'invention peut être constituée de l'une quelconque des solutions aqueuses dites de qualité " industrielle ", " technique ", " alimentaire " ou " FCC ", " pharmaceutique " ou " USP ", accessibles dans le commerce, y compris leurs variantes tamponnées et/ou stabilisées à la chaleur. Il peut s'agir également de solutions aqueuses pouvant contenir de l'acide lactique se présentant en tout ou partie sous forme de sel(s) apte(s) à se dissocier significativement avant et/ou pendant l'étape d'estérification tels que le lactate d'ammonium.

En outre, et bien que cela ne constitue pas la variante préférentielle du procédé selon l'invention, la composition d'acide lactique mise en oeuvre peut déjà, du fait d'un procédé particulier de préparation ou de recyclage, associé ou non au procédé selon l'invention, contenir des traces ou quelques pourcents d'ester(s) d'acide lactique.

Conformément à l'invention ladite composition est transformée, dans une première étape, en une composition oligomérique d'acide lactique et ce, avec élimination d'eau.

De manière avantageuse cette transformation est opérée par simple évaporation, en une ou plusieurs étapes et ce, jusqu'à obtenir une composition oligomérique particulière, à savoir présentant un degré de polymérisation moyen (DPM) compris entre 2 et 30 environ. Une telle composition présente l'avantage, outre de constituer un moyen d'apport d'eau limité en vue de l'étape ultérieure d'estérification, de minimiser le coût de résolution des problèmes de mise en oeuvre, mélange, transport et autres opérations unitaires, rencontrés avec des produits beaucoup plus visqueux tels que les polylactides et autres polymères de hauts poids moléculaires utilisés comme matière première dans le brevet US 5,264,617 précité.

La durée nécessaire à l'obtention, notamment par évaporation, de la composition oligomérique dépend en premier lieu de la teneur en eau initiale de la composition d'acide lactique et des conditions opératoires appliquées lors de cette étape de transformation.

Celle-ci peut, à titre d'exemple, être menée à une température de 100 à 170°C environ et à la pression atmosphérique ou sous un vide plus ou moins poussé, généralement compris entre 20 et 500 millibars (mbar) soit entre 2 000 et 50 000 pascals(pa).

Bien que cela ne soit aucunement obligatoire, cette étape d'oligomérisation peut, en outre, être avantageusement menée en présence d'au moins un catalyseur de transestérification en vue notamment d'accélérer l'élimination de l'eau. Un intérêt remarquable du procédé selon l'invention est d'ailleurs de permettre d'utiliser la même fonction d'un moyen, en l'occurrence la fonction de catalyseur de transestérification d'un composé chimique, à deux moments dudit procédé, à savoir lors de l'étape a) de transformation de l'acide lactique en composition oligomérique puis lors de l'étape b) de mélange de ladite composition avec un alcool en vue d'estérifier l'acide lactique. Ce moyen peut d'ailleurs être de nature identique et être présent en quantités identiques lors de ces deux étapes successives.

En suite de quoi le procédé selon l'invention peut être caractérisé par le fait que ladite étape a) est également menée en présence d'un catalyseur de transestérification, celui-ci étant, de préférence, identique au catalyseur en présence duquel est menée ladite étape b).

Selon une autre variante, le catalyseur de transestérification en présence duquel est menée ladite étape b) a été mis en oeuvre, en tout ou partie, lors de ladite étape a). La notion de " catalyseur de transestérification " n'est ici aucunement limitative et doit être entendue comme incluant toute espèce de nature chimique ou enzymatique apte à catalyser avantageusement l'une au moins des deux étapes a) et b) précitées. Cette notion recouvre en particulier l'ensemble des catalyseurs dits d'estérification ou de transestérification décrits dans l'un ou l'autre des brevets précités et notamment colonne 6 lignes 44-59 du brevet US 5,723,639, ainsi que leurs équivalents fonctionnels. De préférence, le catalyseur de transestérification est un catalyseur acide choisi en particulier dans le groupe comprenant l'acide sulfurique, l'acide chlorhydrique, l'acide phosphorique, l'acide p-toluène sulfonique, l'acide méthane sulfonique, l'acide propane 1,3-disulfonique, les sels acides desdits acides et les résines acides.

Comme indiqué, le procédé selon l'invention n'impose aucunement de mettre en oeuvre des taux de catalyseurs qui soient supérieurs à ceux classiquement utilisés. Ces taux se situent généralement entre 0,01 et 10 %, exprimés en poids sec de catalyseur par rapport au poids sec d'acide lactique, sous forme monomérique, dimérique, oligomérique ou autre, sous forme libre ou non, contenu dans le milieu réactionnel.

Selon une variante du procédé selon l'invention, le taux de catalyseur est inférieur à 1 %, de préférence compris entre 0,01 et 0,9 %. Il peut par exemple être de l'ordre de 0,1 à 0,5 % en vue de la préparation de lactate d'éthyle, ces valeurs étant significativement inférieures à celles décrites dans la littérature précitée pour une telle préparation. Comme précisé, le catalyseur de transestérification peut, ou non, avoir été mis en oeuvre et ce, totalement ou non, dès l'étape a) de transformation de la composition d'acide lactique en composition oligomérique. De manière caractéristique, cette dernière présente un degré de polymérisation moyen (DPM) de 2 à 30 environ, ledit DPM étant calculé selon la formule suivante :

$$DPM = \frac{18}{\left[\frac{100 \times 90{,}08}{T}\right] - 72}$$

où T correspond à la masse d'acide lactique monomère (CH3 - CHOH - COOH ; 90,08 g/mole) contenue dans 100 g sec de composition oligomérique. Cette masse est déterminée après saponification (20 minutes à 100°C) par de la soude en excès, d'un échantillon de poids déterminé (0,1 à 0,3 g) de la composition oligomérique. Après neutralisation du mélange réactionnel, celui-ci est analysé par technique de Chromatographie Liquide Haute Performance (CLHP) avec détection réfractométrique. Cette analyse peut se faire sur une colonne échangeuse de cations de type " SHODEX SH 1011 " en utilisant de l'acide sulfurique N/100 comme éluant. Cette technique permet de déterminer la masse d'acide lactique monomère contenue dans l'échantillon testé et par simple calcul, la masse T contenue dans 100 g de composition oligomérique.

De manière préférentielle, la composition oligomérique d'acide lactique présente un DPM compris entre 2 et 15 environ, et en particulier compris entre 3 et 10 environ.

Ces valeurs de DPM n'excluent bien évidemment aucunement que la composition oligomérique dont l'obtention est revendiquée puisse contenir, de l'état de traces jusqu'à des taux de l'ordre de 10 à 25 % par exemple, des composés tels que de l'acide lactique monomère, du lactide et/ou des polymères d'acide lactique présentant un degré de polymérisation supérieur à la borne supérieure des gammes de DPM revendiquées. Dès après son obtention, la composition oligomérique peut éventuellement être soumise à un traitement de purification, en une ou plusieurs étapes, visant notamment à en abaisser la teneur en l'un au moins de tels composés et/ou en eau résiduelle.

Conformément à la présente invention, la composition oligomérique d'acide lactique, purifiée ou non, est ensuite soumise, en présence d'un catalyseur, à une étape b) de mélange et de réaction avec un alcool en vue d'estérifier tout ou partie de l'acide lactique contenu, sous forme monomérique, dimérique, oligomérique ou polymérique, dans ladite composition. L'alcool mis en oeuvre peut notamment consister en un alcool aliphatique comportant de 1 à 6 atomes de carbone, tels que ceux décrits dans les brevets précités. L'alcool est choisi de préférence dans le groupe comprenant les alcools aliphatiques comportant de 2 à 5 atomes de carbone et encore plus préférentiellement dans le groupe comprenant l'éthanol, le n-butanol et l'isobutanol. Il peut s'agir avantageusement d'éthanol comme il sera exemplifié ci-après.

L'alcool est généralement mis en oeuvre à raison de 1 à 10 moles par mole d'acide lactique monomère contenue dans la composition oligomérique, le nombre de moles d'acide lactique monomère contenues dans ladite composition pouvant être déduit du calcul de masse tel que décrit ci-avant.

De préférence, l'alcool est mis en oeuvre à raison de 1,5 à 5 moles par mole d'acide lactique monomère.

La nature et les quantités de catalyseurs pouvant être présents lors de cette étape b) d'estérification de l'acide lactique par un alcool ont été décrites ci-avant, étant rappelé que tout catalyseur de transestérification a pu être mis en oeuvre, en tout ou partie, dès l'étape a) d'obtention de la composition oligomérique à partir d'acide lactique.

De préférence, la température du milieu réactionnel lors de cette étape b) se situe également entre 100 et 170°C, y compris lorsque l'alcool mis en oeuvre consiste en éthanol.

La Société Demanderesse a trouvé par ailleurs que dans le cas de l'éthanol en particulier, cette température pouvait se situer avantageusement dans une gamme allant de 100°C à 145°C, i.e dans une gamme permettant de mener une réaction d'estérification efficace dans des délais relativement courts, généralement inférieurs à 10 heures et sans risque de racémisation de l'acide lactique et/ou de l'ester.

Par ailleurs, ladite réaction est menée sous simple pression "autogène ", i.e sous la seule pression de vapeur du mélange réactionnel à la température réactionnelle.

Dans ces conditions, le rendement en ester d'acide lactique est généralement supérieur à 85 %.

La composition d'ester d'acide lactique obtenue à l'issue de l'étape b) d'estérification peut être utilisée en l'état, i.e sans étape subséquente de purification.

Selon une première variante, ladite composition est soumise à au moins un traitement de purification en vue d'en éliminer notamment tout catalyseur, tout alcool libre et/ou toute trace d'eau éventuelle qu'elle pourrait contenir. Un tel traitement peut en particulier consister en un traitement de distillation, simple ou multiple.

L'alcool contenu dans la fraction alcoolique issue dudit traitement de distillation peut ensuite être déshydraté, notamment par un traitement de pervaporation ou de déshydratation sur tamis moléculaire puis avantageusement être recyclé au niveau de l'étape b) d'estérification du procédé selon l'invention.

A titre d'exemple, la composition d'ester d'acide lactique, par exemple de lactate d'éthyle, récupérée à l'issue de l'étape b) peut être purifiée par un traitement double de distillation aux fins d'en éliminer en particulier 1) l'éthanol puis 2) le catalyseur et les traces d'acide lactique et/ou de lactoyl lactate d'éthyle (dimère estérifié) éventuellement présentes. La fraction éthanolique ainsi obtenue par distillation peut alors être soumise à un traitement de pervaporation en vue de la déshydrater totalement puis, éventuellement, à une étape de recyclage vers l'étape b) d'estérification.

De même, un traitement double de distillation peut permettre d'obtenir, entre autres, un résidu contenant de l'acide lactique, sous forme libre et/ou estérifiée, et des oligomères d'acide lactique, sous forme libre et/ou estérifiée. Un tel résidu peut également être soumis à une étape de recyclage vers l'étape b) d'estérification.

Les procédés et équipements aptes à être utilisés en vue de la purification, en une ou plusieurs étapes, d'une composition d'ester d'acide lactique obtenue conformément à l'invention ou de la récupération éventuelle de l'alcool sont ceux dont dispose classiquement l'homme de l'art et incluent ceux décrits dans la littérature, en particulier dans les brevets WO 93/15127, US 5,210,296 et US 5,723,639 précités.

En suite de quoi, on dispose désormais d'un nouveau procédé de préparation d'esters d'acide lactique, à la fois simple, efficace et peu coûteux, ledit procédé pouvant avantageusement être mené en continu du fait, entre autres, de la bonne fluidité du produit intermédiaire que constitue la composition oligomérique d'acide lactique telle que décrite, laquelle est généralement déjà liquide à des températures de l'ordre de 80 à 100°C et peut donc être facilement acheminée vers l'étape b) d'estérification.

Le procédé selon l'invention est tout particulièrement adapté à la préparation de lactate d'éthyle, ce produit pouvant avantageusement être utilisé dans les domaines d'activité précités y compris, comme l'a constaté la Demanderesse, dans une utilisation nouvelle de solvant pour la préparation de compositions gélifiées de toutes natures et destinations, en particulier celles contenant un diacétal d'alditol comme le dibenzylidène sorbitol (DBS) et ses dérivés, notamment alcoylés.

EXEMPLE 1

Dans un évaporateur rotatif, on introduit 1 kg d'une composition d'acide lactique consistant en une solution aqueuse d'acide lactique à 88 % et 4 g d'acide sulfurique concentré à 96 %. On transforme ladite composition en chauffant le milieu réactionnel à 130°C et sous vide (100 - 1000 mbars) et ce, pendant une durée de 5 heures.

On obtient ainsi 650 g environ d'une composition oligomérique d'acide lactique, parfaitement fluide. La masse d'acide lactique monomère contenue dans ladite composition puis le degré de polymérisation moyen (DPM) de ladite composition sont calculés comme indiqué précédemment.

Dans le cas présent, on obtient une composition oligomérique d'acide lactique dont le DPM est de 5,9 environ.

Celle-ci est alors mélangée, en continu et sans étape intermédiaire de purification, à de l'éthanol et ce, à raison de 3 moles d'éthanol par mole d'acide lactique monomère contenue dans la composition et sans rajouter de catalyseur de transestérification. Après 8 heures d'estérification à 140°C et sous pression autogène, on récupère la composition de lactate d'éthyle résultante. Le rendement en lactate d'éthyle se situe à une valeur significativement supérieure à 85 %, en l'occurrence 96 %.

La composition de lactate d'éthyle ainsi récupérée est ensuite soumise à un traitement de purification par distillation. En suite de quoi, on obtient une composition de lactate d'éthyle présentant une pureté supérieure à 99 %.

EXEMPLE 2

on évalue l'intérêt de la composition de lactate d'éthyle purifiée obtenue selon l'EXEMPLE 1, comme solvant en vue de la préparation d'une composition gélifiée contenant, entre autres, 2 % en poids de dibenzylidène sorbitol (DBS), 0,5 % d'hydroxypropyl cellulose (HPC), 40,5 % de 3-méthyl 3-méthoxybutanol (MMB), 15 % d'éthanol, 2 % de polyvinylpyrrolidone (PVP), 5 % d'eau, 20 % de parfum et 5 % de diméthicone copolyol (DCP). Le lactate d'éthyle est mis en oeuvre à raison de 10 % du poids de ladite composition. Celle-ci est préparée par mélange à chaud (≈ 90°C) de deux compositions intermédiaires contenant respectivement :

- HPC, MMB, PVP et lactate d'éthyle pour l'une, et
- DBS, éthanol et eau pour l'autre.

Après solubilisation totale du DBS dans le mélange résultant, on y incorpore le parfum, lequel a été préalablement mélangé à la DCP.

Après homogénéisation, on met la composition ainsi obtenue dans un moule en vue d'obtenir un article gélifié de forme précise. On constate que la composition obtenue présente une bonne homogénéité, une bonne transparence et une bonne dureté de gel, supérieure à celle obtenue en substituant le lactate d'éthyle, poids par poids, par du propylène glycol ou du MP diol. En outre, du fait de sa moindre hygroscopicité en regard des autres solvants testés, le lactate d'éthyle permet à la composition gélifiée de perdre du poids de manière plus significative et régulière au cours du temps, ce qui constitue un résultat avantageux lorsque ladite composition est destinée à être utilisée comme gel ambianceur.

EXEMPLE 3

Dans un réacteur double-enveloppe, on introduit 1 kg d'une solution aqueuse d'acide lactique à 88 %. On transforme ladite solution en chauffant le milieu réactionnel à 150°C et sous vide et ce, pendant une durée de 5 heures. On obtient ainsi 690 g environ d'une composition oligomérique d'acide lactique, parfaitement fluide. Dans le cas présent, on obtient une composition oligomérique d'acide lactique dont le DPM est de 3,4. Celle-ci est alors mélangée à de l'éthanol (1240 g) et ce, à raison de 3 moles d'éthanol par mole d'acide lactique monomère et à 4 g d'acide sulfurique concentré à 96 %. Après 8 heures d'estérification à 140°C et sous pression autogène, on récupère la composition de lactate d'éthyle résultante. Le rendement en lactate d'éthyle s'établit à 90 %. La composition de lactate d'éthyle ainsi récupérée peut ensuite être soumise à un traitement de purification par distillation.

Cet exemple montre que l'étape a) conforme au procédé selon l'invention peut être effectuée en l'absence de tout catalyseur de transestérification.

EXEMPLE 4

130 g d'un résidu de distillation de lactate d'éthyle composé notamment de 12 % environ de lactate d'éthyle, de 12 % environ d'acide lactique et d'oligomères d'acide lactique (sous forme libre ou estérifiée), est porté à reflux pendant 4 heures en présence de 1,5 g d'acide sulfurique et 115 g d'éthanol. On obtient ainsi une composition contenant majoritairement (environ 60 % en poids) du lactate d'éthyle, le reste de ladite composition étant principalement composée de dimères d'acide lactique (sous forme libre ou estérifiée).

Cet exemple montre que l'on peut avantageusement envisager, dans le cadre de l'invention, de recycler un résidu de distillation contenant des oligomères d'acide lactique vers l'étape b) d'estérification.

**Revendications**

1. Procédé de préparation d'une composition d'ester d'acide lactique à partir d'une composition d'acide lactique, **caractérisé par le fait qu'**il comprend :

> a) une étape de transformation, avec élimination d'eau, de ladite composition d'acide lactique en une composition oligomérique d'acide lactique présentant un degré de polymérisation moyen (DPM) compris entre 2 et 30,
> b) une étape subséquente de mélange et de réaction de ladite composition oligomérique, purifiée ou non, avec un alcool, en présence d'un catalyseur de transestérification, en vue d'estérifier tout ou partie de l'acide lactique contenu, sous forme monomérique, dimérique, oligomérique ou polymérique, dans ladite composition oligomérique, et
> c) une étape éventuelle de purification de la composition d'ester d'acide lactique ainsi obtenue.

2. Procédé selon la revendication 1 **caractérisé par le fait que** l'étape a) de transformation de la composition d'acide lactique en une composition oligomérique est également menée en présence d'un catalyseur de transestérification, ledit catalyseur étant, de préférence, identique au catalyseur en présence duquel est menée l'étape b) d'estérification.

3. Procédé selon la revendication 2 **caractérisé par le fait que** le catalyseur en présence duquel est menée l'étape b) d'estérification a été mis en oeuvre, en tout ou partie, lors de l'étape a) de transformation, ledit catalyseur étant, de préférence, choisi dans le groupe comprenant l'acide sulfurique, l'acide chlorhydrique, l'acide phosphorique, l'acide p-toluène sulfonique, l'acide méthane sulfonique, l'acide propane 1,3-disulfonique, les sels acides desdits acides et les résines acides.

4. Procédé selon l'une quelconque des revendications 1 à 3 **caractérisé par le fait que** le taux de catalyseur de transestérification présent lors de l'étape b) d'estérification et, éventuellement, lors de l'étape a) de transformation, est inférieur à 1 %, de préférence compris entre 0,01 et 0,9 %, ces pourcentages étant exprimés en poids sec de catalyseur par rapport au poids sec d'acide lactique, sous forme monomérique, dimérique, oligomérique ou autre, sous forme libre ou non, contenu dans le milieu réactionnel.

5. Procédé selon l'une quelconque des revendications 1 à 4 **caractérisé par le fait que** la composition oligomérique d'acide lactique présente un degré de polymérisation moyen (DPM) compris entre 2 et 15, en particulier compris entre 3 et 10.

6. Procédé selon l'une quelconque des revendications 1 à 5 **caractérisé par le fait que** l'alcool mis en oeuvre lors de l'étape b) d'estérification est choisi dans le groupe comprenant les alcools aliphatiques comportant de 2 à 5 atomes de carbone, de préférence dans le groupe comprenant l'éthanol, le n-butanol et l'isobutanol et que la température du milieu réactionnel, lors de ladite étape b), se situe entre 100 et 170°C.

7. Procédé selon la revendication 6 **caractérisé par le fait que** l'alcool consiste en éthanol et que la température du milieu réactionnel se situe entre 100 et 145°C.

8. Procédé selon l'une quelconque des revendications 1 à 7 **caractérisé par le fait que** la composition d'ester d'acide lactique obtenue à l'issue de l'étape b) d'estérification est soumise à au moins un traitement de purification, de préférence un traitement de distillation simple ou multiple.

9. Procédé selon l'une quelconque des revendications 1 à 8 **caractérisé en ce qu'**il est mené en continu.

10. Utilisation de lactate d'éthyle en tant que solvant pour la préparation de compositions gélifiées, en particulier celles contenant un diacétal d'alditol choisi de préférence dans le groupe comprenant le dibenzylidène sorbitol (DBS) et ses dérivés alcoylés.

## Patentansprüche

1. Verfahren zur Herstellung einer Milchsäureesterzusammensetzung, ausgehend von einer Milchsäurezusammensetzung, **dadurch gekennzeichnet, dass** es umfasst:

a) einen Überführungsschritt der Milchsäurezusammensetzung, unter Eliminierung von Wasser, zu einer Milchsäureoligomerzusammensetzung, die einen mittleren Polymerisationsgrad (MPG) zwischen 2 und 30 aufweist,

b) einen nachfolgenden Misch- und Reaktionsschritt der gereinigten oder nicht gereinigten Oligomerzusammensetzung mit einem Alkohol, in der Gegenwart eines Umesterungskatalysators, um die in der Oligomerzusammensetzung in monomerer, dimerer, oligomerer oder poylmerer Form enthaltene Milchsäure vollständig oder partiell zu verestern,

c) gegebenenfalls einen Reinigungsschritt der derart erhaltenen Milchsäureesterzusammensetzung.

2. Verfahren nach Anspruch 1, **dadurch gekennzeichnet, dass** der Überführungsschritt a) der Milchsäurezusammensetzung zu einer Oligomerzusammensetzung ebenfalls in der Gegenwart eines Umesterungskatalysators durchgeführt wird, wobei der Katalysator bevorzugt gleich dem Katalysator ist, in dessen Gegenwart der Veresterungsschritt b) durchgeführt wird.

3. Verfahren nach Anspruch 2, **dadurch gekennzeichnet, dass** der Katalysator, in dessen Gegenwart der Veresterungsschritt b) durchgeführt wird, vollständig oder teilweise während des Überführungsschritts a) verwendet worden ist, wobei der Katalysator bevorzugt ausgewählt wird aus der Gruppe, umfassend Schwefelsäure, Chlorwasserstoffsäure, Phosphorsäure, p-Toluolsulfonsäure, Methansulfonsäure, Propan-1,3-disulfonsäure, den Salzen der Säuren und den Säureharzen.

4. Verfahren nach einem der Ansprüche 1 bis 3, **dadurch gekennzeichnet, dass** die Menge des während des Veresterungsschritts b) und gegebenenfalls während des Überführungsschritts a) vorhandenen Umesterungskatalysators weniger als 1 % beträgt, bevorzugt zwischen 0,01 und 0,9 % liegt, wobei diese Prozentangaben ausgedrückt sind als Trockengewicht des Katalysators bezogen auf das Trockengewicht der im Reaktionsmilieu enthaltenen Milchsäure in monomerer, dimerer, oligomerer oder anderer Form, in freier Form oder nicht-freier Form.

5. Verfahren nach einem der Ansprüche 1 bis 4, **dadurch gekennzeichnet, dass** die Milchsäureoligomerzusammensetzung einen mittleren Polymerisationsgrad (MPG) zwischen 2 und 15, insbesondere zwischen 3 und 10 aufweist.

6. Verfahren nach einem der Ansprüche 1 bis 5, **dadurch gekennzeichnet, dass** der im Veresterungsschritt b) verwendete Alkohol ausgewählt wird aus der Gruppe, umfassend die aliphatischen Alkohole mit 2 bis 5 Kohlenstoffatomen, bevorzugt aus der Gruppe, umfassend Ethanol, n-Butanol und Isobutanol, und dass die Temperatur des Reaktionsmilieus während des Schritts b) zwischen 100 und 170°C liegt.

7. Verfahren nach Anspruch 6, **dadurch gekennzeichnet, dass** der Alkohol aus Ethanol besteht und dass die Temperatur des Reaktionsmilieus während des Schritts b) zwischen 100 und 145°C liegt.

8. Verfahren nach einem der Ansprüche 1 bis 7, **dadurch gekennzeichnet, dass** die aus dem Veresterungsschritt b) erhaltene Milchsäureesterzusammensetzung mindestens einer Reinigungsbehandlung unterzogen wird, bevorzugt einer einfachen oder mehrfachen Destillationsbehandlung.

9. Verfahren nach einem der Ansprüche 1 bis 8, **dadurch gekennzeichnet, dass** es kontinuierlich durchgeführt wird.

10. Verwendung von Ethyllactat als Lösungsmittel für die Herstellung von gelierten Zusammensetzungen, insbesondere denjenigen, welche ein Alditoldiacetal, bevorzugt ausgewählt aus der Gruppe, umfassend Dibenzylidensorbitol (DBS) und dessen alkylierte Derivate, enthalten.

## Claims

1. A process for the preparation of a lactic acid ester composition from a lactic acid composition, **characterized in that** it comprises:

a) a stage of conversion, with removal of water of said lactic acid composition into a lactic acid oligomeric composition exhibiting a mean degree of polymerization (MDP) comprises between 2 and 30,

b) a subsequent stage of mixing and of reaction of said purified or unpurified oligomeric composition with an alcohol, using a transesterification catalyst, in order to esterify all or a portion of the lactic acid present, in a monomeric, dimeric, oligomeric or polymeric form, in said oligomeric composition, and

c) an optional stage of purification of the lactic acid ester composition thus obtained.

2. The process as claimed in claim 1, **characterized in that** stage a) for conversion of the lactic acid composition into an oligomeric composition is also carried out using a transesterification catalyst, said catalyst being preferably identical to the catalyst used for the esterification stage b).

3. The process as claimed in claim 2, **characterized in that** the catalyst used for the esterification stage b) was used, in all or in part, for the conversion stage a), said catalyst being preferably selected from the group comprising sulfuric acid, hydrochloric acid, phosphoric acid, p-toluenesulfonic acid, methanesulfonic acid, propane-1,3-disulfonic acid, the acid salts of said acids and acid resins.

4. The process as claimed in any one of claims 1 to 3, **characterized in that** the amount of transesterification catalyst used for the esterification stage b) and optionally for the conversion stage a) is less than 1%, preferably comprised between 0.01 and 0.9%, these percentages being expressed as dry weight of catalyst with respect to the dry weight of lactic acid, present in the reaction medium, free or not as a monomeric, dimeric, oligomeric or another form.

5. The process as claimed in any one of claims 1 to 4, **characterized in that** the lactic acid oligomeric composition exhibits a mean degree of polymerization (MDP) comprises between 2 and 15, in particular comprises between 3 and 10.

6. The process as claimed in any one of claims 1 to 5, **characterized in that** the alcohol employed for the esterification stage b) is selected from the group comprising aliphatic alcohols comprising from 2 to 5 carbon atoms, preferably from the group comprising ethanol, n-butanol and isobutanol, and **in that** the temperature of the reaction, in said stage b), is between 100 and 170°C.

7. The process as claimed in claim 6, **characterized in that** the alcohol consists of ethanol and **in that** the temperature of the reaction is between 100 and 145°C.

8. The process as claimed in any one of claims 1 to 7, **characterized in that** the lactic acid ester composition obtained from the esterification stage b) is subjected to at least one purification step, preferably a simple or multiple distillation.

9. The process as claimed in any one of claims 1 to 8, **characterized in that** it is carried out continuously.

10. The use of ethyl lactate as a solvent for the preparation of gelled compositions, in particular those comprising an alditol diacetal preferably chosen from the group comprising dibenzylidene sorbitol (DBS) and its alkylated derivatives.